# EUROPÄISCHE PATENTSCHRIFT

(11) **EP 3 273 928 B1**
(45) Veröffentlichungstag und Bekanntmachung des Hinweises auf die Patenterteilung: **05.10.2022**
(21) Anmeldenummer: 16710996.6
(22) Anmeldetag: 22.03.2016
(51) Int. Cl.: A61K 6/15, A61K 6/887

(54) **VERFAHREN ZUR HERSTELLUNG DENTALER PROTHESEN**
METHOD FOR PRODUCING DENTAL PROSTHESES
PROCÉDÉ DE FABRICATION DE PROTHÈSES DENTAIRES

(30) Priorität: 24.03.2015 DE 102015104440
(43) Veröffentlichungstag der Anmeldung: 31.01.2018
(73) Patentinhaber: Kulzer GmbH, 63450 Hanau (DE)
(72) Erfinder: RUPPERT, Klaus, 63477 Maintal (DE); HOHMANN, Alfred, 61389 Schmitten (DE); DEKERT, Stephan, 71273 Wehrheim (DE)
(74) Vertreter: Bendele, Tanja
(86) Internationale Anmeldenummer: PCT/EP2016/056261
(87) Internationale Veröffentlichungsnummer: WO 2016/150962

(56) Entgegenhaltungen:
- EP-A1- 1 444 972
- EP-A1- 1 564 155
- EP-A1- 2 233 123
- EP-A2- 0 995 421
- EP-A2- 1 243 230
- WO-A1-2005/002531
- WO-A1-2011/081976
- WO-A1-2015/006087
- WO-A2-01/43700
- DE-A1- 19 941 829
- DE-U1- 29 624 496
- US-A- 5 554 665
- US-A1- 2006 264 532
- US-A1- 2007 043 142
- US-A1- 2011 315 928
- US-A1- 2012 296 003
- US-B1- 6 180 688
- XU HOCKIN H K ET AL: "Dental glass-reinforced composite for caries inhibition: calcium phosphate ion release and mechanical properties.", JOURNAL OF BIOMEDICAL MATERIALS RESEARCH. PART B, APPLIED BIOMATERIALS FEB 2010, vol. 92, no. 2, February 2010 (2010-02), pages 332-340, ISSN: 1552-4981
- GBURECK U ET AL: "Rheological enhancement of mechanically activated [alpha]-tricalcium phosphate cements", JOURNAL OF BIOMEDICAL MATERIALS RESEARCH - PART B APPLIED BIOMATERIALS 200504 US, vol. 73, no. 1, April 2005 (2005-04), pages 1-6, ISSN: 0021-9304

## Beschreibung

Es wird ein Verfahren zur Herstellung dentaler prothetischer Formteile sowie ein gebrauchsfertiges, polymerisierbares Dentalmaterial zur Herstellung dentaler prothetischer Formteile, wie von Teil- oder Totalprothesen, Brückenteilen, Kronen, vorgeschlagen. Das gebrauchsfertige Dentalmaterial wird vorzugsweise in dem erfindungsgemässen Verfahren eingesetzt. Das gebrauchsfertige Dentalmaterial ist, optional nach dem Mischen des getrennt als zwei Pasten vorliegenden Dentalmaterials mit einem in den Pasten aufgeteilten Initiatorsystem und ansonsten im Wesentlichen identischen Dentalmaterial, ohne Anquellzeit sofort zu prothetischen Formteilen aushärtbar bzw. polymerisierbar. Offenbart wird ein Kit umfassend das gebrauchsfertige, polymerisierbare Dentalmaterial, sowie die Verwendung des Kits und ein Verfahren zur Herstellung dentaler prothetischer Formteile. Das Dentalmaterial umfasst einen Initiator oder ein Initiatorsystem zur Redoxreaktion (Kalthärtung) oder Heisshärtung.

Prothesenwerkstoffe auf (Meth-)acrylat-Basis bestehen üblicherweise aus einem Pulver-Flüssigkeitssystem, welches durch Vermischen der beiden Komponenten in einem definierten Verhältnis nach einer Warte- und Anquellzeit einen verarbeitungsfähigen Teig ergibt. Dieser Teig wird dann in den Hohlraum, meist eine Küvette umfassend eine Negativform der herzustellenden Prothese, für die herzustellende Prothese gegossen, gepresst oder injiziert und anschliessend bei Kaltpolymerisaten durch eine Redoxreaktion (z.B. Amin-Peroxid oder Barbitursäure), bei Heisspolymerisaten durch den thermischen Zerfall z.B. eines Peroxides, die Polymerisation ausgelöst.

Ein wesentlicher Nachteil des bekannten Verfahrens und der bekannten Zusammensetzungen bestehen darin, dass die beiden üblichen Hauptkomponenten der Dentalmaterialien, PMMA-Pulver und MMA-Flüssigkeit, durch intensives Rühren vermischt werden. Dieses Mischen dauert in der Regel ca. 30 sec.. An das Mischen schliesst sich dann eine Anquellzeit an, in der die Mischung zu einem verarbeitungsfähigen, pastösen Teig anquillt (auch als "putty" oder"dough" bezeichnet). Diese Anquellzeit nach dem Mischen dauert üblicherweise bei Heisspolymerisaten ca. 10 bis 15 Minuten und bei Kaltpolymerisaten ca. 1 bis 3 min.. Neben dem erheblichen Zeitaufwand besteht hierbei auch immer die Gefahr, dass durch das Rühren Luftblasen in den Werkstoff eingebracht werden und damit die mechanische Festigkeit der Prothese reduziert wird.

Eine weitere potentielle Fehlerquelle ist eine Nichteinhaltung des Mischungsverhältnisses durch den Anwender, welches die Qualität des Prothesenwerkstoffes ebenfalls negativ beeinflussen kann. Einerseits kann die mechanische Festigkeit abnehmen, andererseits kann ein erhöhter Restmonomergehalt mit den damit für den Patienten verbundenen Gesundheitsgefährdungen auftreten.

Ferner muss nach dem Vermischen von Pulver und Flüssigkeit eine bestimmte Wartezeit eingehalten werden, damit das Methylmethacrylat die PMMA-Perlen anlösen und -quellen kann. Erst durch diesen Quellprozess entsteht ein verarbeitungsfähiger Teig. Diese Unterbrechung des Arbeitsablaufes ist im Dentallabor störend und unerwünscht.

Da der Quellprozess auch von der Umgebungstemperatur abhängig ist, wird die Konsistenz des Teiges vom Verarbeiter typischerweise mit der Hand überprüft, hierdurch können Hautallergien und Unverträglichkeiten entstehen bzw. gefördert werden.

Ferner kommt es im Dentallabor durch das offene Vermischen der Pulver- mit der Flüssigkeitskomponente typischerweise zu einer erheblichen Belastung mit MMA-Dämpfen sowie Staubteilchen aus PMMA-Pulver.

US5554665 offenbart ein Verfahren zur Herstellung von Dentalwerkstoffen mittels pastenförmiger kaltpolymerisierender und Licht-härtender Zusammensetzungen. DE29624496U1 offenbart ein gefülltes und polymerisierbares Dentalmaterial. EP1564155A1 betrifft einen Zweikammer-Druckbehälter zum Verpacken und Ausbringen fließfähiger hochviskoser oder fließfähiger faserhaltiger Dentalmaterialien. DE19941829A1 offenbart ein autipolymerisierbare Dentalzusammensetzung. US2011/0315928A1 offenbart ein fließfähiges, polymerisierbares Dentalmaterial. EP1243230A2 offenbart ein Verfahren zur Herstellung einer Prothese sowie Prothesenwerkstoffe zur Durchführung des Verfahrens.

Eine Aufgabe der Erfindung bestand daher darin, ein Verfahren und ein Dentalmaterial zur Herstellung von Dentalprothesen anzugeben, bei dem die oben aufgeführten Nachteile bei der Herstellung von Prothesenwerkstoffen entfallen oder minimiert werden. Somit war eine Aufgabe, die Entwicklung eines Verfahrens, das eine Belastung und Kontamination mit Monomer-Dämpfen und Staubteilchen bei der Herstellung dentaler prothetischer Formteile zuverlässig vermeidet und vorzugsweise zugleich die Bearbeitungszeiten reduziert. Des Weiteren bestand die Aufgabe ein Dentalmaterial anzugeben, das vorzugsweise frei von MMA ist und im Wesentlichen vollständig durchpolymerisiert und daher nach der Polymerisation keinen Restmonomergehalt mehr aufweist. Zudem sollte ein Dentalmaterial bereitgestellt werden, das ohne eine Anquellzeit angewendet werden kann und keine qualitativen Verschlechterungen hinsichtlich mechanischer Festigkeit wie z.B. Biegefestigkeit, E-Modul oder der Bruchzähigkeit auftritt. Zudem muss das Dentalmaterial weiterhin lagerfähig sein.

Erfindungsgemäss wird eine Aufgabe durch das erfindungsgemässe Verfahren nach Anspruch 1 gelöst. Weitere bevorzugte Ausführungsformen sind in den Unteransprüchen sowie in der Beschreibung offenbart.

Gegenstand der Erfindung ist ein Verfahren zur Herstellung dentaler prothetischer Formteile, insbesondere einer dentalen Total- oder Teilprothese, umfassend die Schritte:
- Verwenden, vorzugsweise Bereitstellen, eines gebrauchsfertigen, polymerisierbaren Dentalmaterials welches eine Viskosität von 5.000 mPa·s bis 20.000 mPa·s bestimmt bei Raumtemperatur (ca. 23 °C) aufweist und eine Mischung von Monomeren und optional Polymeren umfasst, die als eine einzige Phase in der Mischung vorliegen, als mindestens eine Paste, insbesondere mit einer Viskosität von 1.000 mPa·s bis 75.000 mPa·s, in mindestens einer oder zwei Kartusche(n),
- Austragen des gebrauchsfertigen Dentalmaterials, optional unter Mischen des Dentalmaterials, und
- Einbringen des Dentalmaterials in eine Negativform zur Herstellung von mindestens einem dentalen prothetischen Formteil, und
- optional Polymerisieren des Dentalmaterials, wobei die Gesamtzusammensetzung des Dentalmaterials 100 Gew.-% umfasst und die Mischung zu
   (1)25 bis 90 Gew.-% mindestens ein Urethan(meth)acrylat, insbesondere Urethandimethacrylat, oder eine Mischung umfassend mindestens zwei Urethan(meth)acrylate;
   (2)0,5 bis 40 Gew.-% mindestens ein Urethan-Gruppen freies Di(meth)acrylat umfasst propoxyliertes Neopentylglykoldiacrylat, Triethylenglycoldimethacrylat, Diethylenglycoldimethacrylat Decandioldi(meth)acrylat, Dodecandioldi-(meth)acrylat, ethoxylierte/ propoxylierte Bisphenol-A-di(meth)acrylate oder eine Mischung umfassend mindestens zwei Di(meth)acrylate;
   (3)0 bis 40 Gew.-% mindestens ein Urethan-Gruppen freies Tri-, Tetra- oder höher funktionelles (Meth)acrylat oder eine Mischung umfassend mindestens zwei der vorgenannten (Meth)acrylate,
   (4)0,1 bis 60 Gew.-% anorganischen, amorphen Füllstoff oder eine Mischung anorganischer Füllstoffe, ausgewählt aus pyrogener Kieselsäure oder ein anorganischer Füllstoff umfassend Fällungskieselsäuren, Dentalgläser, Aluminosilicatgläsern, Fluoroaluminosilicatgläsern, Bariumaluminiumsilicat, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis, Glasfasern und/oder Kohlenstofffasern sowie Mischungen umfassend mindestens einen der vorgenannten Füllstoffe, und/oder
   (5)0,01 bis 5 Gew.-% eines Initiators oder Initiatorsystems zur Heisspolymerisation umfasst.

Sowie vorzugsweise Erhalten eines dentalen prothetischen Formteils, insbesondere einer dentalen Total- oder Teilprothese, die eine Biegefestigkeit von größer gleich 60 [MPa] aufweist, vorzugsweise größer gleich 62 [MPa], weiter bevorzugt größer gleich 64 [MPa], insbesondere bis 100 [MPa], und/oder ein E-Modul von größer gleich 1500 [MPa] aufweist, insbesondere größer gleich 1900 [MPa], bevorzugt größer gleich 2100 [MPa], weiter bevorzugt größer gleich 2200 [MPa], insbesondere bis 3500 [MPa]. Die vorgennannten Parameter für die Biegefestigkeit und den E-Modul werden insbesondere nach DIN ISO 20795-1: 2013-06, bestimmt.

Das offenbarte gebrauchsfertige, polymerisierbare Dentalmaterial ist kein lichthärtendes polymerisierbares Dentalmaterial, sondern ein heisshärtendes Dentalmaterial oder ein autopolymerisierendes Dentalmaterial, insbesondere ein autopolymerisierendes 2K-Dentalmaterial, und vorzugsweise ist das Dentalmaterial ein PMMA und/oder MMA freies Dentalmaterial.

Nach einer bevorzugten Variante des Verfahrens wird als gebrauchsfertiges, polymerisierbares Dentalmaterial eine Mischung von Monomeren und optional Polymeren, die als eine einzige Phase in der Mischung vorliegen eingesetzt, wobei die Gesamtzusammensetzung des Dentalmaterials 100 Gew.-% umfasst und die Mischung umfasst zu
(1) 25 bis 90 Gew.-%, insbesondere 20 bis 40 Gew.-% oder 40 bis 90 Gew.-% mindestens ein Urethan(meth)acrylat, insbesondere Urethandimethacrylat, oder eine Mischung umfassend mindestens zwei Urethan(meth)acrylate, insbesondere zu 60 bis 85 Gew.% oder in einer Alternative als Komponenten (i) 40 bis 75 Gew.-% und als Komponenten (ii) 5 bis 30 Gew.-%, insbesondere zu 5 bis 20 Gew.-%, bevorzugt 10 bis 15 Gew.-%,
(2) 0,5 bis 40 Gew.-%, insbesondere 0,5 bis 30 Gew.-% mindestens ein Urethan-Gruppen freies Di(meth)acrylat oder einer Mischung umfassend mindestens zwei Di(meth)acrylate, insbesondere 5 bis 20 Gew.-%, bevorzugt 5 bis 15 Gew-%, besonders bevorzugt um 10 Gew.-% mit einer Schwankungsbreite von +/- 5 Gew.-%, bevorzugt +/- 2 Gew.-%;
(3) 0 bis 40 Gew.-%, insbesondere 5 bis 40 Gew.-% mindestens ein Urethan-Gruppen freies Tri-, Tetra- oder höher funktionelles (Meth)acrylat oder einer Mischung umfassend mindestens zwei der vorgenannten (Meth)acrylate, insbesondere 5 bis 20 Gew.-%, bevorzugt 5 bis 15 Gew.-%, besonders bevorzugt um 10 Gew.-% vorzugsweise mit einer Schwankungsbreite von +/- 5 Gew.-%, besonders bevorzugt +/-2 Gew.-%;
optional können zusätzlich zur Mischung, um 100 Gew.-% der Dentalzusammensetzung einzustellen, weitere Komponenten wie anorganische Füllstoffe, Stabilisatoren, Aktivatoren, Pigmente oder Initiatoren eingesetzt werden. Durch Zugabe der weiteren Komponenten kann aus der einphasigen Mischung eine Dispersion gebildet werden.

Dabei ist es nach einer Alternative bevorzugt, wenn das Dentalmaterial als Paste A und Paste B getrennt in einer Kartusche (Doppelkammerkartusche) oder zwei Kartuschen vorliegt und beim Austragen gemischt wird. Vorzugsweise erfolgt das Mischen mit einem zwischen Kartusche und Negativform vorgesehenen Statikmischer. Das Austragen des Dentalmaterials kann manuell oder automatisch erfolgen. Nach einer weiteren bevorzugten Alternative liegt das Dentalmaterial in einem Kit in zwei getrennten Kartuschen, in einer Kartusche als Paste A und der zweiten Kartusche als Paste B, vor oder in einer Kartusche (Doppelkammerkartusche) getrennt voneinander als Paste A und B vor.

Des Weiteren ist es bevorzugt, dass die Kartusche(n) und die Einlassöffnung der Negativform zum Einbringen des Dentalmaterials in die Negativform aneinander befestigbar sind. Dies kann mittels dem Fachmann bekannten Massnahmen, wie aneinanderschrauben, verspannen oder dergleichen erfolgen. Vorzugsweise ist die Verbindung zwischen Kartusche(n) und Einlassöffnung nach aussen abgedichtet, so dass kein Dentalmaterial an der Verbindungstelle austreten kann.

Ferner kann nach einer bevorzugten Verfahrensvariante das Einbringen in die Negativform pneumatisch erfolgen. Dies kann automatisch oder manuell mittels einer Austragspistole bzw. Dispersers erfolgen. Nach einer Ausführungsvariante ist das Verfahren ein pneumatisches Injektionsverfahren oder alternativ ein manuelles. Bevorzugt ist ein pneumatisches Verfahren in dem als Negativform bspw. eine konventionelle Küvette eingesetzt wird. Besonders bevorzugt erfolgt das Einbringen in die Negativform bei einem Druck von 0,5 bis 10 bar, vorzugsweise 1 bis 10 bar, insbesondere 1,1 bis 5 bar, bevorzugt von 1,1 bis 2,5 bar, weiter bevorzugt um 1,5 bis 2,5 oder um 2,0 bar mit +/- 0,5 bar. Dabei weist die Kartusche einen Statikmischer auf, der in der Einlassöffnung der Negativform festgelegt ist. Die Polymerisation kann vorzugsweise bei Raumtemperatur oder bei 40 bis 70 °C, vorzugsweise um 55 °C +/- 5 °C erfolgen.

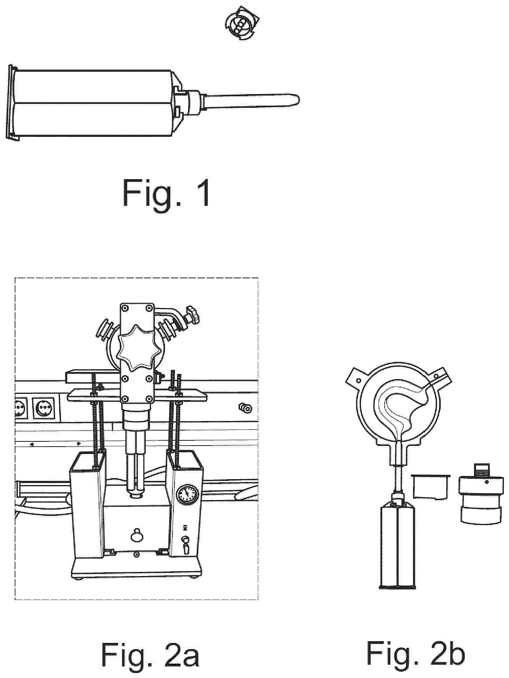

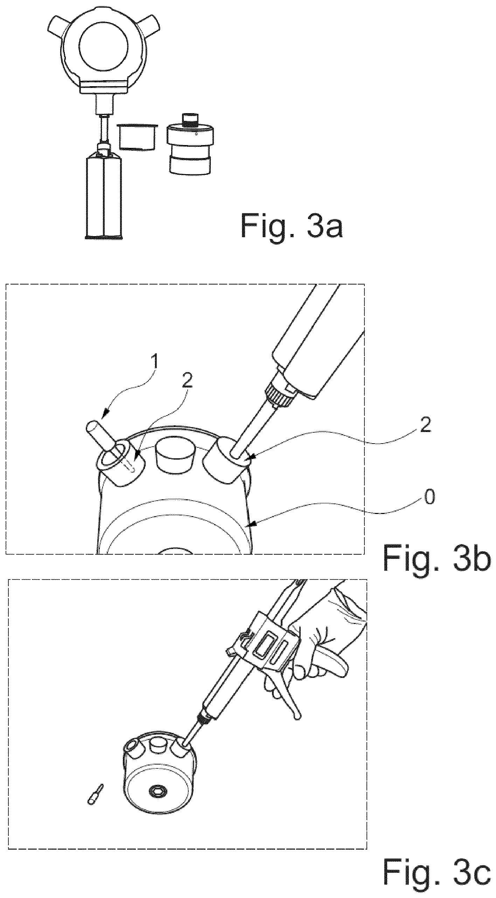

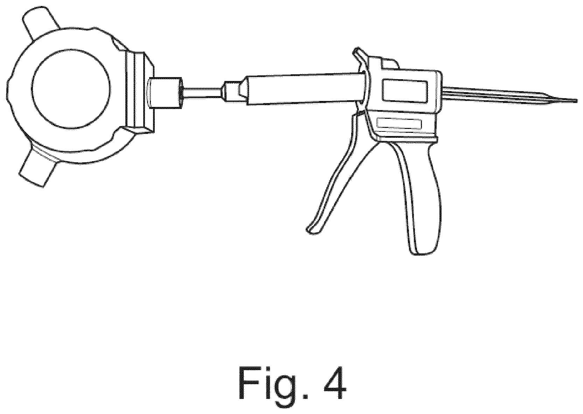

Das Einbringen des Dentalmaterials erfolgt vorzugsweise auch während der Polymerisation, um ggf. den bei der Polymerisation auftretenden Schrumpf auszugleichen. Auf diese Weise werden sehr passgenaue prothetische Formteile, insbesondere ohne spätere Randspalten, erhalten. Die Polymerisation kann mittels Heisshärtung oder über eine Autopolymerisation erfolgen. Das erfindungsgemässe Verfahren wird in den Figuren 1 bis 4 veranschaulicht.

Nach dem erfindungsgemässen Verfahren wird vorzugsweise polymerisiert und anschliessend das mindestens eine dentale prothetische Formteil erhalten. Vorzugsweise wird in dem Verfahren das nachfolgend beschriebene Dentalmaterial oder ein Kit eingesetzt. Darüber hinaus ist jedes polymerisierbare Dentalmaterial, insbesondere in einer Kartusche, mit einer bestimmten Viskosität geeignet in dem erfindungsgemässen Verfahren eingesetzt zu werden.

Das offenbarte Dentalmaterial ermöglicht die Durchführung des erfindungsgemässen Verfahrens ohne die sonst üblichen Wartezeiten beim Vermischen der bekannten Prothesenmaterialien aus dem Stand der Technik umfassend MMA und PMMA-Perlen. Nach der Erfindung werden vorzugsweise keine lichthärtenden polymerisierbaren Dentalmaterialien in dem Verfahren eingesetzt. Daher ist ebenso Gegenstand der Erfindung ein Verfahren in dem das Dentalmaterial oder ein Kit verwendet wird.

Erfindungsgemäss wird die Aufgabe durch ein Verfahren gelöst in dem pasten- oder cremeförmiges, polymerisierbares Dentalmaterial bzw. Prothesenwerkstoff, bei denen sämtliche Komponenten in einer verarbeitungsfähigen und zeitlich stabilen Konsistenz vorliegen und keine oder nur noch geringe Anquell- oder Anlösevorgänge stattfinden. Ferner ist die Konsistenz des unpolymerisierten, gebrauchsfertigen Dentalmaterials, insbesondere der Pasten A und/oder B zeitlich stabil.

Das offenbarte Dentalmaterial ist sofort gebrauchsfertig und weist eine verarbeitungsfähige Konsistenz auf. In einer besonders bevorzugten Ausführungsform der Erfindung liegt das Dentalmaterial als eine Paste A und eine Paste B vor, die sich lediglich durch die Initiatoren oder die Initiatorkomponenten eines Initiatorsystems unterscheiden.

Im Falle eines Kaltpolymerisates weisen beide Komponenten des Dentalmaterials vorzugsweise eine sehr ähnliche Zusammensetzung des Dentalmaterials auf und unterscheiden sich in der Regel durch den anwesenden Initiator.

Der offenbarte 2K-Prothesenwerkstoff wird vorzugsweise in eine Doppelkammerkartusche (1:1 Doppelkammerkartusche) oder eine Koaxial-Kartusche (z.B. Peeler von Sulzer Mixpac) abgefüllt, das Vermischen der beiden Komponenten und damit der Start der Polymerisation erfolgt beim Fördern durch einen Statik-Mischer am Kartuschenauslass.

Neben der Verwendung des kaltpolymerisierenden Prothesenwerkstoffes direkt aus dem Statik-Mischer, z.B. für Reparaturen und Unterfütterungen, Prothesensättel oder zum Einfliessen in eine Giessküvette, kann der Dentalwerkstoff inkl. Packmittel auch zum direkten Injizieren in eine Küvette, z.B. mittels des Palajet-Systems oder einer Dispensing-Gun, verwendet werden.

Im Falle eines pastenförmigen Heisspolymerisates kann der offenbarte Prothesenwerkstoff in eine 1K-Kartusche abgefüllt von dort direkt über ein Anschlussrohr in eine Küvette injiziert werden. Um die Packmittel möglichst identisch zu halten, kann das Heisspolymerisat alternativ dazu auch in eine Doppelkammerkartusche abgefüllt werden, in diesem Fall umfassen die Kammern der Kartusche quantitativ und qualitativ identisches Dentalmaterial. Typischerweise enthalten beide Kammern der Kartusche Material aus der gleichen Farbe. Optional dazu kann eine Kammer auch farbloses Material enthalten und die farbgebende Komponente befindet sich in der anderen Kammer. Durch Mischen der beiden Komponenten entsteht dann die Endfarbe.

Das Dentalmaterial kann als Heisspolymerisat vorliegen und kann durch Erhitzen auspolymerisiert werden. Vorzugsweise wird das Heisspolymerisat oberhalb 70 °C, vorzugsweise bei grösser gleich 95 °C, auspolymerisiert. Liegt das Dentalmaterial als Auto-oder Kaltpolymerisat vor, kann es durch Vermischen der Pasten A und B auspolymerisiert werden.

Durch die gebrauchsfertige Formulierung des Prothesenwerkstoffes und das entsprechend geeignete Packmittel entfällt zudem die Berührung von allergieauslösendem MMA mit der Hand und auch die Staubbelastung durch PMMA-Pulver sowie die Belastung mit MMA-Dämpfen ist eliminiert.

Offenbart wird ein polymerisierbares, insbesondere gebrauchsfertiges Dentalmaterial, bevorzugt zur Herstellung dentaler prothetischer Formteile, das eine Viskosität von 1.000 mPa·s bis 75.000 mPa·s aufweist, insbesondere weist es eine Viskosität von 1.000 mPa·s bis 50.000 mPa·s, bevorzugt von 5.000 bis 20.000 mPa·s., besonders bevorzugt von 10.000 bis 15.000 mPa·s, auf, und umfasst eine Mischung von Monomeren und optional Polymeren die als eine einzige Phase in dieser Mischung vorliegen. Optional kann das polymerisierbare Dentalmaterial 0,1 bis 50 Gew.-% mindestens eines anorganischen Füllstoffs umfassen, der in der Phase der Mischung dispergiert vorliegt, wobei die Gesamtzusammensetzung des Dentalmaterials 100 Gew.-% beträgt. Die Viskosität wird vorzugsweise bestimmt bei Raumtemperatur, circa 23 °C und vorzugsweise einer Scherrate von 1/10 sec mit einem üblichen Viskosimeter.

Als eine einzige Phase im Sinne der Erfindung gilt ein polymerisierbares Dentalmaterial, dessen reine Mischung von Monomeren und optional Polymeren - ohne anorganischen Füllstoff - als eine einzige flüssige Phase vorliegt. Erfindungsgemäss liegen die Monomere und Polymere als eine einzige flüssige Phase ohne innere Phasengrenzen vor. Insoweit liegen die Monomere und Polymere nicht als eine Dispersion vor. Nach Zugabe der anorganischen Füllstoffe bildet sich aus der einen Phase der Mischung der Monomere und Polymere eine Dispersion.

Nach einer offenbarten Variante umfasst das polymerisierbare Dentalmaterial eine Mischung von Monomeren und optional Polymeren, die als eine einzige Phase in der Mischung vorliegen, wobei die Gesamtzusammensetzung des Dentalmaterials 100 Gew.-% umfasst und die Mischung umfasst zu
(1) 25 bis 90 Gew.-%, insbesondere 20 bis 40 Gew.-% oder 40 bis 90 Gew.-% mindestens ein Urethan(meth)acrylat, insbesondere Urethandimethacrylat, oder eine Mischung umfassend mindestens zwei Urethan(meth)acrylate, insbesondere zu 60 bis 85 Gew.-% oder in einer Alternative als Komponenten (i) 40 bis 75 Gew.-% und als Komponenten (ii) 5 bis 30 Gew.-%, insbesondere zu 5 bis 20 Gew.-%, bevorzugt 10 bis 15 Gew.-%,
(2) 0,5 bis 40 Gew.-%, insbesondere 0,5 bis 30 Gew.-% mindestens ein Urethan-Gruppen freies Di(meth)acrylat oder einer Mischung umfassend mindestens zwei Di(meth)acrylate, insbesondere 5 bis 20 Gew.-%, bevorzugt 5 bis 15 Gew.-%, besonders bevorzugt um 10 Gew.-% mit einer Schwankungsbreite von +/- 5 Gew.-%, bevorzugt +/- 2 Gew.-%;
(3) 0 bis 40 Gew.-%, insbesondere 5 bis 40 Gew.-% mindestens ein Urethan-Gruppen freies Tri-, Tetra- oder höher funktionelles (Meth)acrylat oder einer Mischung umfassend mindestens zwei der vorgenannten (Meth)acrylate, insbesondere 5 bis 20 Gew.-%, bevorzugt 5 bis 15 Gew.-%, besonders bevorzugt um 10 Gew.-% vorzugsweise mit einer Schwankungsbreite von +/- 5 Gew.-%, besonders bevorzugt +/- 2 Gew.-%;
optional können zusätzlich zur Mischung, um 100 Gew.-% der Dentalzusammensetzung einzustellen, weitere Komponenten wie anorganische Füllstoffe, Stabilisatoren, Aktivatoren, Pigmente oder Initiatoren eingesetzt werden. Durch Zugabe der weiteren Komponenten kann aus der einphasigen Mischung eine Dispersion gebildet werden.

Nach einer offenbarten Ausführungsform umfasst das Dentalmaterial als weitere Komponenten
(4) 0,1 bis 60 Gew.-%, insbesondere 0,1 bis 30 Gew.-%, vorzugsweise 0,1 bis 10 Gew.-% oder alternativ 10 bis 50 Gew.-% anorganischen, amorphen Füllstoff oder eine Mischung anorganischer Füllstoffe, und/oder
(5) 0,01 bis 5 Gew.-% eines Initiators, insbesondere eines radikalischen Initators, oder ein Initiatorsystem zur Heiss- oder Kaltpolymerisation bzw. Kalthärtung, wobei die Gesamtzusammensetzung des Dentalmaterials 100 Gew.-% beträgt.

Die offenbarten Dentalmaterialien können, vorzugsweise 0,01 bis 2 Gew.-%, mindestens ein Farbpigment oder eine Mischung von Farbpigmenten in der Gesamtzusammensetzung umfassen.

Nach einer offenbarten Ausführungsvariante umfasst das Dentalmaterial als das mindestens eine Urethan(meth)acrylat (1), insbesondere Urethandimethacrylat, oder eine Mischung umfassend mindestens zwei Urethan(meth)acrylate, vorzugsweise zu 10 bis 75 Gew.-% in der Mischung,
(i) mindestens ein Urethandi(meth)acrylat, insbesondere Bis(methacryloxy-2-ethoxycarbonylamino)-alkylen, besonders bevorzugt ist Bis(methacryloxy-2-ethoxycarbonylamino)-alkylen, mit Alkylen von 2 bis 15 C-Atomen, insbesondere ein C9-Alkylen, und vorzugsweise zu 5 bis 30 Gew.-% in der Mischung,
(ii) mindestens ein Urethantri(meth)acrylat, insbesondere aliphatischer Polyester eines Triurethantriacrylats, aliphatisches Urethantriacrylat.

Das mindestens eine Urethan(meth)acrylat oder eine Mischung von Urethan(meth)acrylaten umfasst mindestens ein Urethan(meth)acrylat, Urethandi(meth)acrylat, Urethantri(meth)-acrylat oder ein mehrfach funktionelles Urethan(meth)acrylat, wie Urethandimethacrylat, Urethandi(meth)acrylat aus der Umsetzung eines a, ω-funktionalisierten Alkyldiisocyanat OCN-(CH₂)ₙ-CNO mit n = 2 bis 20, insbesondere mit n = 9 mit einem HO-funktionellen Acrylat, insbesondere Hydroxyehtylacrylat, insbesondere ein Urethandimethacrylat, bevorzugt ein Bis(methacryloxy-2-ethoxycarbonylamino)-alkylen, aliphatischer Polyester eines Triurethantriacrylats, Diurethanacrylat Oligomer, Alkyl-funktionelle Urethandimethacrylat Oligomere, Aromatisch-funktionalisierte Urethandimethacrylat Oligomere, aliphatische ungesättigte Urethanacrylate, Bis(methacryloxy-2-ethoxycarbonylamino) substituierter Polyether, aromatische Urethandiacrylat Oligomere, aliphatische Urethandiacrylat Oligomere, monofunktionelle Urethanacrylate, aliphatische Urethandiacrylate, hexafunktionelle aliphatische Urethanharze, aliphatisches Urethantriacrylat, UDMA, aliphatisches Urethanacrylat Oligomer, ungesättigtes aliphatisches Urethanacrylat.

Besonders bevorzugt ist Bis(methacryloxy-2-ethoxycarbonylamino)-alkylen, mit Alkylen von 2 bis 15 C-Atomen, insbesondere einem C9-Alkylen,

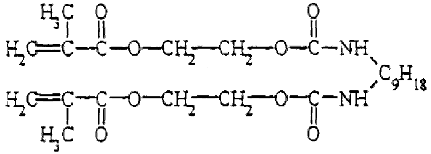

Das mindestens eine Urethan-Gruppen freie Di(meth)acrylat oder Mischung von Urethan-Gruppen freiem Di(meth)acrylat umfassend mindestens zwei Di(meth)acrylate, umfasst vorzugsweise Di-, Tri- oder Tetraethylenglykoldi(meth)acrylat, propoxyliertes Neopentylglykoldiacrylate, Alkyldioldi(meth)acrylat mit C2 bis C15 in der Alkyl-Gruppe, Decandioldi(meth)acrylat, Dodecandioldi(meth)acrylat, Hexyldecandioldi(meth)acrylat, Butandioldi(meth)acrylat, Ethylenglycoldi(meth)acrylat, Polyethylenglycol-di(meth)acrylate, ethoxylierte/propoxylierte Bisphenol-A-di(meth)acrylate und/oder HDDMA (1,6-Hexanedioldimethacrylat), Bisphenol-A-di(meth)acrylate (BPDMA), 1,4-Butandioldimethacrylat (1,4-BDMA), Bis-GMA-Monomer Bisphenyl-A-Glycidyl-Methacrylat (ein Additionsprodukt aus Methacrylsäure und Bisphenol-A-diglycidyl-ether), Methacrylatbasiertes di-funktionelles eine Polyisocyanurate-Gruppe umfassende Monomere und/oder Diethylenglykoldi(meth)acrylat. Die folgenden difunktionellen Monomere können auch als Verdünnungsmittel (dünnflüssige Acrylate wie Triethylenglycoldimethacrylat (TEGDMA) und Diethylenglycol-dimethacrylat (DEGMA)) zugesetzt werden, bspw. zur Einstellung der Viskosität. Besonders bevorzugt sind Decandioldi(meth)acrylat, Dodecandioldi(meth)acrylat und/oder ethoxylierte/propoxylierte Bisphenol-A-di(meth)acrylate.

Das mindestens eine Urethan-Gruppen freie Tri-, Tetra- oder höher funktionelles (Meth)acrylat oder eine Mischung umfassend mindestens zwei der vorgenannten (Meth)acrylate (3) umfasst vorzugsweise Tri- oder Tetraethylenglykoldi(meth)acrylat, Trimethylolpropan-tri(meth)acrylat, Methacrylat basiertes tri- und/oder tetra-funktionelle Polyisocyanurate-Gruppen umfassende Monomere, tris(2-Hydroxyethyl)-isocyanurattriacrylat und/oder Pentaerythritol-tetraacrylat.

Eine bevorzugte Mischung umfasst Derivate von Glycoldi(meth)acrylaten, wie propoxyliertes Neopentylglykoldiacrylate, ein Urethandiacrylat wie Bis(methacryloxy-2-ethoxycarbonylamino)-alkylen, mit Alkylen von 2 bis 15 C-Atomen, insbesondere einem C9-Alkylen sowie Triethylenglycoldimethacrylat (TEGDMA) und Diethylenglycol-dimethacrylat (DEGMA) zugesetzt werden, bspw. zur Einstellung der Viskosität. Besonders bevorzugt sind Decandioldi(meth)acrylat, Dodecandioldi(meth)acrylat und/oder ethoxylierte/propoxylierte Bisphenol-A-di(meth)acrylate.

In dem offenbarten gebrauchsfertigen Dentalmaterial wird vorzugsweise als anorganischer Füllstoff oder als Mischung anorganischer Füllstoffe vorzugsweise mindestens ein amorpher Füllstoff eingesetzt. Vorzugsweise wird pyrogene Kieselsäure oder ein anorganischer Füllstoff umfassend Fällungskieselsäuren, Dentalgläser, wie Aluminosilicatgläser oder Fluoroaluminosilicatgläser, Bariumaluminiumsilicat, Strontium-silicat, Strontium-borosilicat, Lithiumsilicat, Lithiumaluminiumsilicat, Schichtsilikate, Zeolithe, amorphe sphärische Füller auf Oxid- oder Mischoxidbasis, insbesondere Mischoxide von SiO₂ und ZrO₂, Glasfasern und/oder Kohlenstofffasern sowie Mischungen umfassend mindestens einen der vorgenannten Füllstoffe, bevorzugt sind pyrogene Kieselsäure und Mischoxide von SiO₂ und ZrO₂, verwendet.

Vorzugsweise enthält das offenbarte polymerisierbare Dentalmaterial keinen Gehalt an organischen Polymeren, wie ein Polymeth-acrylsäure-methylester (PMMA), Polymethylacrylsäureethylester (PMEA), Methacrylsäuremethylester (MMA) und/oder Ethylacrylsäure-ester (EMA) sowie vernetzte Polymere und/oder Co-Polymere. Zweckmässig kann der Gehalt der organischen Polymere von 0,0 Gew.-%, insbesondere 0,001 Gew.-% bis 5,0 Gew.-% in der Gesamtzusammensetzung aufweisen. Der Gehalt an Methylmethacrylat (MMA) und/oder Ethylmethacrylat (EMA) liegt vorzugweise ebenfalls bei 0,0 Gew.-%. Zweckmässig können 0,0001 bis 0,1 Gew.-% MMA und/oder EMEA enthalten sein, wobei das polymerisierbare Dentalmaterial vorzugweise völlig frei von MMA und/oder EMA ist.

Des Weiteren wird Kit offenbart, umfassend ein polymerisierbares Dentalmaterial zur Herstellung von dentalen prothetischen Formteilen, wobei das Dentalmaterial als Paste mit einer Viskosität von 1.000 mPa·s bis 75.000 mPa·s, insbesondere von 1.000 mPa·s bis 50.000 mPa·s, vorzugsweise mit einer Viskosität von 5.000 bis 20.000 mPa·s, bevorzugt von 5.000 bis 15.000 mPa·s, in einer Kartusche vorliegt.

In dem polymerisierbaren Dentalmaterial liegen die monomeren und optional polymeren Komponenten in einer Phase vor, insbesondere als flüssige Phase, vorzugsweise als eine einzige Phase (homogene Phase), bevorzugt liegt die Phase als eine einzige flüssige Phase vor, in die optional anorganische Füllstoffe dispergiert sind. Das Vorliegen einer Phase kann mikroskopisch festgestellt werden. Offenbart wird ein pastenförmiges, sofort gebrauchsfertiges Dentalmaterial als ein Einkomponenten- oder Zweikomponenten-Dentalmaterial. Das pastenförmige Dentalmaterial liegt bei Zugabe von anorganischen Füllstoffen als gebrauchsfertige Dispersion und ohne Zugabe dieser Füllstoffe als eine gebrauchsfertige einphasige Phase vor.

Des Weiteren wird ein Kit offenbart, umfassend das gebrauchsfertige, polymerisierbare Dentalmaterial in mindestens einer Kartusche oder in zwei Kartuschen, insbesondere ist die Kartusche eine Koaxialkartusche oder eine Doppelkammerkartusche. Weiter ist es bevorzugt, wenn die Kartusche derart ausgebildet ist, dass sie für die Anwendung in einem Injektionsverfahren zur Herstellung von dentalen prothetischen Formteilen geeignet ist. Vorzugsweise liegt das Dentalmaterial als eine gebrauchsfertige und lagerfähige Paste mit einer Viskosität von 1.000 mPa·s bis 75.000 mPa·s vor. Besonders bevorzugte liegt die Viskosität im Bereich von 1.000 mPa·s bis 50.000 mPa·s, vorzugsweise 1.000 bis 20.000 mPa·s, bevorzug von 5.000 bis 15.000 mPa·s.

Ferner wird ein Kit offenbart, aufweisend eine Paste A und eine Paste B, wobei die Pasten A und B jeweils unabhängig eine Mischung von Monomeren und optional Polymeren umfassen, die jeweils als eine einzige Phase vorliegen, insbesondere als flüssige Phase. Dabei ist es insbesondere weiter bevorzugt, wenn die (a) Paste A eine Viskosität von 1.000 mPa·s bis 75.000 mPa·s aufweist, insbesondere eine Viskosität von 1.000 mPa·s bis 50.000 mPa·s, vorzugsweise 1.000 bis 15.000 mPa·s, bevorzug von 2.000 bis 10.000 mPa·s und umfasst (a1) mindestens ein Urethan(meth)acrylat, (a2) mindestens ein Urethan-Gruppen freies Di(meth)acrylat, (a3) mindestens ein Urethan-Gruppen freies Tri-, Tetra- oder höher funktionelles (Meth)acrylat, (a4) optional mindestens einen anorganischen Füllstoff und, wobei die (b) Paste B eine Viskosität von 1.000 mPa·s bis 75.000 mPa·s aufweist, insbesondere eine Viskosität von 1.000 mPa·s bis 50.000 mPa·s, vorzugsweise 5.000 bis 20.000 mPa·s, bevorzug von 5.000 bis 17.500 mPa·s und umfasst (b1) mindestens ein Urethan(meth)acrylat,
(b2) mindestens ein Urethan-Gruppen freies Di(meth)acrylat, (b3) mindestens ein Urethan-Gruppen freies Tri-, Tetra- oder höher funktionelles (Meth)acrylat, (b4) optional mindestens einen anorganischen Füllstoff, wobei optional der Füllstoff (a4) und/oder (b4) der in der Phase der Mischung dispergiert vorliegt,
und wobei mindestens eine der Pasten A und/oder B als Komponente (a5) bzw. (b5) mindestens einen Gehalt eines (a5) radikalischen Initiators oder radikalischen Initiatorsystems zur Heiss- oder Kalthärtung umfasst, und wobei die Gesamtzusammensetzung 100 Gew.-% beträgt.

Des Weiteren wird ein Kit offenbart, umfassend Pasten A und B, wobei die Pasten in den folgenden Alternativen vorliegen können: (i) wobei mindestens eine der Pasten A und/oder B als Komponente (a5) bzw. (b5) mindestens einen Gehalt eines radikalischen Initiators umfassend eine Peroxid-Verbindung und optional mindestens ein Amin umfasst oder (ii) wobei (a5) und (b5) als Komponenten eines Redoxsystems auf die Pasten A und B aufgeteilt sind und erst mit dem Mischen der Pasten A und B als Komponente (a5) bzw. (b5) mindestens je einen Bestandteil eines Redoxsystems umfasst. Beispielsweise liegen ein Peroxid und ein Amin voneinander getrennt jeweils in einer der Pasten A und B vor. Ebenso kann (i) mindestens eine der Pasten A und/oder B als Komponente (a5) bzw. (b5) mindestens einen Gehalt eines radikalischen Initiators eine Azo-Verbindung umfassen, wobei die Verbindung auf die Pasten A und B aufgeteilt sein kann.

Die die Pasten A und B unterscheiden sich nur durch den Initiatorkomplex.

Ferner kann es bevorzugt sein, wenn Paste A mindestens einen Gehalt eines radikalischen Initiators umfassend eine Peroxid-Verbindung und optional mindestens ein Amin umfasst, und Paste B einen Aktivator umfasst, wie ein aromatisches Amin-Derivat. Bevorzugte Verbindungen sind exemplarisch nachfolgend aufgeführt. Gleichfalls bevorzugt kann optional eine der Pasten A oder B als Komponente (a5) bzw. (b5) mindestens eine KupferVerbindung, mindestens eine Halogenid-Quelle und mindestens eine Barbitursäure(n) oder Barbitursäure-Derivate umfassen. Bevorzugte Komponenten (a5) und (b5) sind nachfolgend aufgeführt. Dem Fachmann ist bekannt, dass ebenfalls weitere übliche Initiatoren/-systeme in der gebrauchsfertigen, polymerisierbaren Dentalzusammensetzung eingesetzt werden können.

Offenbart wird ein Kit umfassend eine gebrauchsfertige, polymerisierbare Dentalzusammensetzung, umfassend eine (a) Paste A mit einer Viskosität von 1.000 mPa·s bis 75.000 mPa·s, insbesondere von 1.000 mPa·s bis 50.000 mPa·s, vorzugsweise mit einer Viskosität von 1.000 bis 15.000 mPa·s, bevorzugt von 2.000 bis 10.000 mPa·s, wobei die Paste eine Mischung aufweist, die umfasst zu
(a1) 25 bis 80 Gew.-%, insbesondere 20 bis 40 Gew.-% oder 40 bis 80 Gew.-% mindestens ein Urethan(meth)acrylat, insbesondere Urethandimethacrylat, oder eine Mischung umfassend mindestens zwei Urethan(meth)acrylate,
(a2) 0,5 bis 40 Gew.-%, insbesondere 0,5 bis 30 Gew.-% mindestens ein Urethan-Gruppen freies Di(meth)acrylat, oder eine Mischung umfassend mindestens zwei Di(meth)acrylate, (a3) 0 bis 40 Gew.-%, insbesondere 5 bis 40 Gew.-% mindestens ein Urethan-Gruppen freies Tri-, Tetra- oder höher funktionelles (Meth)acrylat oder eine Mischung umfassend mindestens zwei der vorgenannten (Meth)acrylate,
(a4) optional 0,1 bis 60 Gew.-%, insbesondere 0,1 bis 45 Gew.-%, vorzugsweise 0,1 bis 10 Gew.-% anorganischer Füllstoff, insbesondere mindestens ein amorpher anorganischer Füllstoff, sowie eine
(b) Paste B mit einer Viskosität von 1.000 mPa·s bis 75.000 mPa·s, insbesondere von 1.000 mPa·s bis 50.000 mPa·s, vorzugsweise mit einer Viskosität von 5.000 bis 20.000 mPa·s, bevorzugt von 5.000 bis 17.500 mPa·s, wobei die Paste B eine Mischung aufweist, die umfasst zu
(b1) 25 bis 80 Gew.-%, insbesondere 20 bis 40 Gew.-% oder 40 bis 80 Gew.-% mindestens ein Urethan(meth)acrylat, insbesondere Urethandimethacrylat, oder eine Mischung umfassend mindestens zwei Urethan(meth)acrylate,
(b2) 0,5 bis 40 Gew.-%, insbesondere 0,5 bis 30 Gew.-% mindestens ein Urethan-Gruppen freies Di(meth)acrylat, oder eine Mischung umfassend mindestens zwei Di(meth)acrylate, (b3) 0 bis 40 Gew.-%, insbesondere 5 bis 40 Gew.-% mindestens ein Urethan-Gruppen freies Tri-, Tetra- oder höher funktionelles (Meth)acrylat oder eine Mischung umfassend mindestens zwei der vorgenannten (Meth)acrylate,
(b4) optional 0,1 bis 60 Gew.-%, insbesondere 0,1 bis 45 Gew.-%, vorzugsweise 0,1 bis 10 Gew.-% anorganischer Füllstoff, bevorzugt mindestens ein amorpher, anorganischer Füllstoff,
und wobei optional mindestens eine der Pasten A und/oder B als Komponente (a4) bzw. (b4) oder beide Pasten A und B mindestens 0,01 bis 5 Gew.-% eines (a5) radikalischen Initiators oder radikalischen Initiatorsystems zur Heiss- oder Kalthärtung umfassen, wobei die Gesamtzusammensetzung der jeweiligen Paste A und B 100 Gew.-% beträgt.

Nach einer offenbarten Ausführungsform umfasst die Paste A als (a4) ein Peroxid (Oxidationsmittel), insbesondere Cumenhydroperoxyd, und die Paste B umfasst als (b4) mindestens eine Verbindung mit einem ionischen Halogenatom und mindestens einem Kupfersalz oder Kupferkomplex, insbesondere Kupfer-(II)-chlorid, mindestens eine Barbitursäure oder Thiobarbitursäure, oder, insbesondere einen Acetylthioharnstoff, und/oder mindestens eine Chloridquelle, wie Methyltrioctylammoniumchlorid, und optional mindestens einen Polymerisationsregler, wie Y-Terpinen. Dem Fachmann ist klar, dass die Komponenten (b4) ebenso als (a4) und (a4) als (b4) eingesetzt werden können.

Die Pasten A und B liegen bevorzugt jeweils getrennt in einer Kartusche vor und werden vorzugsweise etwa 1 zu 1 gemischt. Des Weiteren ist es bevorzugt, wenn die Paste A und Paste B bezüglich der Komponenten (a1) bis (a4) und (b1) bis (b4) eine im Wesentlichen quantitativ und qualitativ identische Zusammensetzung aufweisen, insbesondere beträgt die quantitative Abweichung maximal 10 Gew.-% der jeweiligen Verbindungen. Optional kann das gebrauchsfertige Dentalmaterial auch in einer Kartusche mit einer Kammer vorliegen.

Zur Einstellung der ästhetischen Erscheinung können dem Dentalmaterial übliche Pigmente zugemischt werden.

Ein offenbartes Kit umfasst eine Kartusche, die einen elektronischen Sensor aufweist. Der Sensor dient zur Erkennung der Kartusche in einem Fördersystem, wie beispielsweise dem Palajet-System, in das die Kartusche einsetzbar ist. Der Sensor kann ebenso ausgestaltet sein, um in anderen Fördersystemen erkannt zu werden.

Ferner wird ein gebrauchsfertiges, polymerisierbares Dentalmaterial offenbart, das vorzugsweise im Dentalmaterial oder in einer der Pasten (A) oder (B) oder in (A) und (B) zusätzlich mindestens einen oder mehrere Stoff(e) aus den Gruppen der Pigmente, Stabilisatoren, Regler, antimikrobiellen Additive, UV-Absorber, Thixotropiermittel enthält. Solche Additive werden - wie auch Pigmente, Stabilisatoren und Regler - in eher geringen Mengen eingesetzt, z. B. insgesamt zu 0,01 bis 3,0 Gew.-%, besonders bevorzugt enthält das Dentalmaterial 0,01 bis 1,0 Gew.-% der vorgenannten Stoffe bezogen auf die Gesamtmasse des Dentalmaterials. Geeignete Stabilisatoren sind z. B. Hydrochinonmonomethylether oder 2,6-Di-tert.-butyl-4-methylphenol (BHT).

Die folgenden Initiatoren und/oder Initiatorsysteme für die Auto- oder Kaltpolymerisation können umfassen a) mindestens einen Initiator, insbesondere mindestens ein Peroxid, insbesondere LPO: Dilauroylperoxid, BPO: Dibenzoylperoxid, t-BPEH: tert.-Butylper-2-ethylhexanoat, DTBP: Di-tert.-butylperoxid, und b) mindestens einen Aktivator, insbesondere mindestens ein aromatisches Amin, wie N,N-Dimethyl-p-toluidin, N,N-Dihydroxyethyl-p-toluidin und/oder p-Dibenzylaminobenzoesäurediethylester oder c) mindestens ein Initiatorsystem ausgewählt aus Redoxsystemen, insbesondere eine Kombinationen ausgewählt aus Dibenzoylperoxid und Dilauroylperoxid mit Aminen ausgewählt aus N,N-Dimethyl-p-toluidin, N,N-Dihydroxyethyl-p-toluidin und p-Dimethylaminobenzoesäurediethylester oder ein Redoxsystem umfassend ein Peroxid, und ein Reduktionsmittel ausgewählt aus Ascorbinsäure, Ascorbinsäurederivat, Barbitursäure oder ein Barbitursäurederivat, Sulfinsäure, Sulfinsäurederviat, besonders bevorzugt ist ein Redoxsystem umfassend
(i) Barbitursäure oder Thiobarbitursäure oder ein Barbitursäure- oder Thiobarbitursäurederivat und (ii) mindestens ein Kupfersalz oder Kupferkomplex und
(iii) mindestens eine Verbindung mit einem ionischen Halogenatom, besonders bevorzugt ist ein Redoxsystem umfassend 1-Benzyl-5-Phenylbarbitursäure, Kupferacetylacetonat und Benzyldibutylammoniumchlorid. Besonders bevorzugt wird die Polymerisation im 2-Komponenten Dentalmaterial über ein Barbitursäurederivat gestartet. In einer Alternative kann das polymerisierbare Dentalmaterial vorzugsweise frei von Peroxiden sein, während es in einer anderen Alternative als Reparaturwerkstoff, Herstellung von Unterfütterungen von Prothesen und Erweiterungen von Prothesen zur schnelleren Aushärtung Peroxid umfassen kann. Die Komponente (i) kann in Paste A und die Komponenten (ii) und (iii) können in der Paste B vorliegen oder umgekehrt.

Zur Heisshärtung können Azoverbindungen, wie AIBN (2',2'-Azobis(isobutyronitril) und/oder zur Strahlenhärtung Campherchinon optional mit Aminen ausgewählt aus N,N-Dimethyl-p-toluidin, N,N-Dihydroxyethyl-p-toluidin und p-Dimethylaminobenzoesäurediethylester oder auch mit weiteren Photoinitiatoren zur Dualhärtung eingesetzt werden.

Nach einer bevorzugten Verfahrensvariante werden die Paste A und die Paste B, insbesondere die Pasten A und B des offenbarten Kits umfassend eine Kartusche, im Gewichtsverhältnis von 1 : 50 bis 50 : 1, insbesondere im Gewichtsverhältnis 1 : 2 bis 2 : 1, besonders bevorzugt von etwa 1 : 1 gemischt, insbesondere mit einer Schwankungsbreite von plus/minus 0,5, bevorzugt plus/minus 0,25.

Das Mischen der Pasten A und B kann mittels eines Statikmischers an der Auslassöffnung einer Doppelkammerkartusche erfolgen. Gleichfalls kann das Mischen mit üblichen Mischgeräten, beispielsweise einem statischen Mischer oder einem dynamischen Mischer erfolgen.

Ebenso wird ein pigmentiertes, auspolymerisiertes Dentalmaterial offenbart. Ferner wird ein unpigmentiertes, auspolymerisiertes Dentalmaterial offenbart.

Ein Verfahren zur Herstellung dentaler prothetischer Formteile, in dem aus einem Kit umfassend das gebrauchsfertige, polymerisierbare Dentalmaterial, optional als Paste A und B, in mindestens einer Kartusche, zwei Kartuschen oder mehreren Kartuschen, optional das Dentalmaterial unter Mischen ausgetragen wird und in eine Negativform zur Herstellung von mindestens einem dentalen prothetischen Formteil eingebracht wird und optional unmittelbar polymerisiert wird. Vorzugsweise werden Einwegkartuschen verwendet.

Dabei können mehrere Kartuschen verwendet werden, um beispielsweise mittels unterschiedlich farbig eingestellter Pasten und vorzugsweise mittels vorabdefinierter Mischungsverhältnisse der Pasten definierte Farben/Schichten über die Zudosierung der Pasten zu erhalten. Mit dem erfindungsgemässen Verfahren können dentale Brücken, Brückenteile, Prothesenbasisteile, Zähne, Verblendungen etc. hergestellt werden.

Ferner ist Gegenstand der Erfindung ein Verfahren zur Herstellung dentaler prothetischer Formteile, in dem aus dem Kit das gebrauchsfertige, polymerisierbare Dentalmaterial als Paste A und B in zwei getrennten Kartuschen optional unter Mischen ausgetragen wird und in eine Negativform zur Herstellung von mindestens einem dentalen prothetischen Formteil eingebracht wird und optional unmittelbar, insbesondere innerhalb von 2 bis 3 Minuten, die Polymerisation gestartet wird. Vorzugsweise wird das polymerisierbare Dentalmaterial in einem Statik-Mischer auf der Kartusche homogen gemischt, insbesondere erfolgt das homogene Vermischen während der Injektion in eine Negativform.

Des Weiteren ist es bevorzugt, wenn das Dentalmaterial oder die Pasten A und B mittels eines pneumatisch oder elektrisch oder magnetisch betriebenen Injektionsgerätes gemischt und direkt in eine Negativform, insbesondere eine Küvette, injiziert werden. Weitere Vorteile des offenbarten Dentalmaterials zeigen sich darin, dass das gebrauchsfertige Dentalmaterial mittels eines handbetriebenen Austraggerätes gemischt und direkt in eine Negativform, insbesondere eine Küvette, injiziert werden kann. Alternativ kann das gebrauchsfertige Dentalmaterial in einer Kartusche oder Doppelkammerkartusche vorliegen, die einen elektronischen Baustein enthält, welcher durch ein Mischsystem, beispielsweise das Palajet-System, erkannt wird und die Verarbeitung des Materials im Mischsystem, wie dem Palajet freigibt.

Ein weiterer Gegenstand der Erfindung ist ein Verfahren unter Verwendung einer gebrauchsfertigen Dentalzusammensetzung oder eines Kits zur Herstellung von dentalen prothetischen Formteilen, Teilen dentaler Prothesen, wie dentaler Teilprothese, dentale Totalprothesen, orthopädischen Prothesen oder Teilen davon, künstlichen Zähnen, Veneers, Inlays, Onlays, Superstrukturen, dentalen Trägerstrukturen, Brücken, Kronen, Unterfütterungen, Prothesensätteln, Knochenprothesen, Gelenkprothesen, Revisions-Total-Gelenk-Endoprothesen und/oder Spacern, insbesondere in einem manuellen oder pneumatischen Injektionsverfahren, vorzugsweise in einem pneumatischen Verfahren unter einem Druck von 0,5 bis 10 bar, vorzugsweise bei 1 bis 5 bar, weiter bevorzugt bei 1,5 bis 5 bar oder bei 2 bis 5 bar.

Bevorzugt ist ein Verfahren , indem die Dentalzusammensetzung nach dem Mischen oder beim Austragen und optional Mischen aus der Kartusche unmittelbar in eine Küvette oder Negativform zur Herstellung mindestens eines dentalen prothetischen Formteils eingebracht und polymerisiert wird.

Ein besonderer Vorteil der gebrauchsfertigen, polymerisierbaren Dentalmaterialien ist es, dass anders als bei üblichen dentalen Materialien, insbesondere von dentalen Prothesenmaterialien, keine Anquellzeit mehr notwendig ist.

Vorteilhaft entspricht die Menge des Dentalmaterials oder der Pasten A und B in den Kartuschen jeweils der Menge, die zur Herstellung einer Teil- oder Totalprothese benötigt wird. Zur Herstellung einer Totalprothese werden üblicherweise 20 bis 30 g benötigt.

### Ausführungsbeispiele:

**Tabelle 1: Rezeptur Kaltpolymerisierender Prothesenwerkstoff**

| (Angaben in Gew.-%) | | |
|---|---|---|
| | Komponente A \| | Komponente B |
| Urethandimethacrylat | 62% | 62 % |
| Aliphatischer Polyester Triurethanacrylat | 13 % | 13 % |
| Dodecandioldimethacrylat | 5% | 5% |
| Ethoxyliertes Bisphenol-A- Dimethacrylat | 5% | 5% |
| Alkoxyliertes Pentaerythrltol tetraacrylat | 10% | 10% |
| Pyrogene Kieselsäure | 4% | 4% |
| BHT | < 0,5 % | < 0,5 % |
| Cu(II)Cl₂ | 0,001 % | X |
| Methyltrioctylammoniumchlorid | 0,2 % | X |
| PBS (Phenyl-Benzyl-Barbitursäure) | X | 1 % |
| BBS (Butyl-Barbitursäure) | X | 0,2 % |

Eine Anquellzeit der Mischung ist nicht mehr notwendig. Die Dentalmischung ist sofort gebrauchsfertig.

**Tabelle 2: Gegenüberstellung der Verarbeitungszeiten**

| | Erfindungsgemäss | PalaXpress (nicht erfindungsgemäss) |
|---|---|---|
| Abwiegen | 0 | 30 sec. |
| Mischen | 0 | 30 sec. |
| Anquellen | 0 | 3 min. |
| Injizieren | 10 min. | 10 min. |
| Polymerisieren | 30 min. | 30 min. |

**Tabelle 3: Gegenüberstellung der Eigenschaften des polymerisierbaren Dentalmaterials bei Verarbeitung im Mischaggregat und im Statikmischer einer Doppelkartusche (Angaben in Gew.-%)**

| Mischverhältnis | Beispiel 1 | | Beispiel 2 | |
|---|---|---|---|---|
| | | | (Zusammensetzung wie Beispiel 1) | |
| | Komponente A | Komponente B | Komponente A | Komponente B |
| | 1:1 | | 1:1 | |
| Propoxyliertes Neopentylglykoldiacrylate | 20,000 | 20,000 | 20,000 | 20,000 |
| UDMA | 30,000 | 30,000 | 30,000 | 30,000 |
| TEDMA | 9,000 | 9,000 | 9,000 | 9,000 |
| nanoteiliges SiO₂ | 40,000 | 39,500 | 40,000 | 39,500 |
| | | | | |
| Cu-II-Cl₂ | | 0,100 | | 0,100 |
| MethyltrioctylAmmoniumchlorid | | 0,170 | | 0,170 |
| Y-Terpinen | | 0,230 | | 0,230 |
| Cumenehydroperoxyd | 1,000 | | 1,000 | |
| Acetyltioharnstoff | | 1,000 | | 1,000 |
| ges. | 100,00 | 100,00 | 100,00 | 100,00 |

**Tabelle 3: Fortsetzung**

| | | Mischen der Pasten | | Mischen der Pasten | |
|---|---|---|---|---|---|
| | | Paste A | Paste B | Paste A | Paste B |
| Biegefestigkeit | > 60[MPa] | 64,9 | | 66,9 | |
| E - Modul | > 1500[MPa] | 2463 | | 2240 | |
| Aushärtung | Verarbeitung über Hausschild^{[1]} | x | | | |
| | Verarbeitung durch Statikmischer^{[2]} | | | x | |
| | Drucktopf Palamat elite 55°C 20 min | x | | x | |

| | | | | | |
|---|---|---|---|---|---|
| ^{[1]} Mischung der Komponenten A und B nach dem Rotationsmischprinzip in Mischaggregat (Speedmixer 150); ^{[2]} Mischen durch Statikmischer der Doppelkartusche | | | | | |

Die gebrauchsfertigen Pasten A besitzen eine Viskosität von etwa 7600 mPa·s (bestimmt bei einer Scherrate von 1/10 sec), die Pasten B weisen eine Viskosität von etwa 14.100 mPa·s unter den genannten Bedingungen auf. Eine 1: 1 Mischung der beiden Pasten A und B weist eine Viskosität von etwa 12.900 mPa·s auf. Das polymerisierbare Dentalmaterial erfüllt die DIN ISO 20795-1: 2013-06, wenn das Prothesenmaterial nach beiden Verfahren gemischt wird. Somit kann über das erfindungsgemässe Verfahren, insbesondere mit einem polymerisierbaren, gebrauchsfertigen Dentalmaterial nach der Erfindung mit einer Viskosität von 2.000 mPa·s bis 15.000 mPa·s, auf einfache und wirtschaftlichere Art und Weise ein gebrauchsfertiges Dentalmaterial zur Herstellung von Prothesen bereitgestellt werden. Wie oben dargestellt vermindert das erfindungsgemässe Verfahren die Anzahl der Arbeitsschritte und die aufgewendete Zeit bei der Herstellung von Prothesen.

Packmittel + Mischer: Die beiden Komponenten A und B werden in eine handelsübliche Doppelkammerkartusche, welche im Verhältnis 1:1 mischt, abgefüllt. Diese Kartuschen werden z.B. von den Firmen Sulzer Mixpac oder Ritter angeboten. Der Statikmischer sollte möglichst kurz sein und einen Aussendurchmesser von ca. 8 mm aufweisen.

In den Figuren ist die Verwendung dargestellt. Gemäss Figur 1 ist eine Doppelkammerkartusche mit dem polymerisierbaren Dentalmaterial befüllt. Die Figuren 3a, 3b und 3c zeigen die Anwendung in einem Handinjektionsverfahren zur Herstellung von prothetischen Formteilen, in dem das Dentalmaterial unmittelbar in die Giessküvette durch den Statikmischer eingefüllt werden kann, um in der Küvette (Fig. 3b: Giessküvette, Fig. 4 Palajet-Küvette) auszupolymerisieren.

Pneumatisches Injektionsverfahren (Palajet): Der auf die Doppelkammerkartusche aufgeschraubte Statikmischer wird in die Injektionsöffnung einer Palajetküvette eingeführt, wie in den Figuren 2a und 2b dargestellt. Nach dem Fixieren der Küvette im Palajet und Abdichten der Injektionsöffnung wird das Dentalmaterial aus der Kartusche in die Küvette injiziert. Während der Polymerisation des Dentalwerkstoffes in der Küvette wird weiterhin Material aus der Kartusche nachgefördert, um den Polymerisationsschrumpf zu kompensieren. Hierdurch ergeben sich sehr passgenaue Prothesen. Entsprechend der in den Figuren dargestellten sofortigen Verwendbarkeit des gebrauchsfertigen, polymerisierbaren Dentalmaterials kann die Zeit zur Herstellung der Prothesen für den Anwender deutlich reduziert werden. Zudem wird ein direkter Kontakt mit der Haut oder ein Einatmen flüchtiger Komponenten mit dem Dentalmaterial vermieden.

## Patentansprüche

1. Verfahren zur Herstellung dentaler prothetischer Formteile, umfassend die Schritte
- Verwenden eines gebrauchsfertigen, polymerisierbaren Dentalmaterials, welches eine Viskosität von 5.000 mPa·s bis 20.000 mPa·s bestimmt bei Raumtemperatur, ca. 23 °C aufweist und eine Mischung von Monomeren umfasst, die als eine einzige Phase in der Mischung vorliegen, als mindestens eine Paste in mindestens einer Kartusche,
- Austragen des gebrauchsfertigen Dentalmaterials, optional unter Mischen des Dentalmaterials, und
- Einbringen des Dentalmaterials in eine Negativform zur Herstellung von mindestens einem dentalen prothetischen Formteil, und
- optional Polymerisieren des Dentalmaterials,
wobei die Gesamtzusammensetzung des Dentalmaterials 100 Gew.-% umfasst und die Mischung zu
(1) 25 bis 90 Gew.-% mindestens ein Urethandimethacrylat, oder eine Mischung umfassend mindestens zwei Urethan(meth)acrylate;
(2) 5 bis 40 Gew.-% mindestens ein Urethan-Gruppen freies Di(meth)acrylat umfasst propoxyliertes Neopentylglykoldiacrylat, Triethylenglycoldimethacrylat, Diethylenglycoldimethacrylat, Decandioldi(meth)acrylat, Dodecandioldi-(meth)acrylat, ethoxylierte/propoxylierte Bisphenol-A-di(meth)acrylate oder eine Mischung umfassend mindestens zwei Di(meth)acrylate;
(3) 0 bis 40 Gew.-% mindestens ein Urethan-Gruppen freies Tri-, Tetra- oder höher funktionelles (Meth)acrylat oder eine Mischung umfassend mindestens zwei der vorgenannten (Meth)acrylate, und des Weiteren
(4) 0,1 bis 60 Gew.-% anorganischen, amorphen Füllstoff oder eine Mischung anorganischer Füllstoffe, ausgewählt aus pyrogener Kieselsäure oder ein anorganischer Füllstoff umfassend Fällungskieselsäuren, Dentalgläser, Aluminosilicatgläsern, Fluoroaluminosilicatgläsern, Bariumaluminiumsilicat, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis, Glasfasern und/oder Kohlenstofffasern sowie Mischungen umfassend mindestens einen der vorgenannten Füllstoffe, und/oder
(5) 0,01 bis 5 Gew.-% eines Initiators oder Initiatorsystems zur Heisspolymerisation
umfasst.

2. Verfahren nach Anspruch 1, **dadurch gekennzeichnet, dass** das Dentalmaterial als Paste A und Paste B getrennt in einer Kartusche oder zwei Kartuschen vorliegt und beim Austragen gemischt wird.

3. Verfahren nach Anspruch 1 oder 2, **dadurch gekennzeichnet, dass** das Einbringen in die Negativform pneumatisch erfolgt.

4. Verfahren nach einem der Ansprüche 1 bis 3, **dadurch gekennzeichnet, dass** während der Polymerisation weiteres Dentalmaterial in die Negativform eingebracht wird.

5. Verfahren nach einem der Ansprüche 1 bis 4, **dadurch gekennzeichnet, dass** die dentalen prothetischen Formteile umfassen Teile dentaler Prothesen, dentale Totalprothesen, orthopädische Prothesen oder Teile davon, künstliche Zähne, Veneers, Inlays, Onlays, Superstrukturen, dentale Trägerstrukturen, Brücken, Kronen, Unterfütterungen, Prothesensätteln, Knochenprothesen, Gelenkprothesen, Revisions-Total-Gelenkendoprothesen und/oder Spacer.

6. Verfahren nach einem der Ansprüche 1 bis 4, **dadurch gekennzeichnet, dass** das Dentalmaterial nach dem Mischen oder beim Austragen und optional Mischen aus der Kartusche unmittelbar in eine Negativform oder Küvette zur Herstellung mindestens eines dentalen prothetischen Formteils eingebracht und polymerisiert wird.

## Claims

1. Method for the production of dental prosthetic moulded parts, comprising the steps of
- using a ready for use, polymerisable dental material having a viscosity of 5000 mPa·s to 20.000 mPa·s determined at room temperature, about 23 °C and comprising a mixture of monomers, being present as single phase in the mixture, as at least one paste in at least one cartridge,
- discharging the ready for use dental material, optionally by mixing the dental material, and
- charging the dental material into a negative mould for the production of at least one dental prosthetic moulded part, and
- optionally, polymerising the dental material,
wherein the total composition of the dental material is 100 % by weight and, wherein the composition comprises
(1) 25 to 90 % by weight at least one urethane dimethacrylate, or a mixture comprising at least two urethane (meth)acrylates,
(2) 5 to 40 % by weight at least one di(meth)acrylate without urethane groups or a mixture comprising at least two di(meth)acrylates comprising propoxylated neopentylglycol diacrylate, triethylene glycol dimethacrylate, diethylene glycol dimethacrylate, decanediol di(meth)acrylate, dodecanediol di(meth)acrylate, ethoxylated/propoxylated bisphenol-A di(meth)acrylate or a mixture comprising at least two of the afore-mentioned di(meth)acrylates,
(3) 0 to 40 % by weight at least one tri-, tetra- or higher functional (meth)acrylate without urethane groups or a mixture comprising at least two of the afore mentioned (meth)acrylates, und
(4) 0.1 to 60 % by weight inorganic, amorphous filler or a mixture of inorganic fillers selected from pyrogenic silicic acid or an inorganic filler comprising precipitated silicic acid, dental glasses, aluminosilicate glasses or fluoroaluminosilicate glasses, barium aluminium silicate, strontium silicate, strontium borosilicate, lithium silicate, lithium aluminium silicate, phyllosilicates, zeolites, amorphous spherical fillers on oxide or mixed oxide basis, glass fibres and/or carbon fibres, as well as mixtures comprising at least one of the afore-mentioned fillers, and/or,
(5) 0.01 to 5 % by weight of an initiator or initiator system for hot-polymerisation.

2. Method according to claim 1, **characterised in that** the dental material is separated as paste A and paste B in one cartridge or in two cartridges and is mixed when discharging.

3. Method according to claim 1 or 2, **characterised in that** charging into the negative mould ensues pneumatically.

4. Method according to any one of the claims 1 to 3, **characterised in that** during polymerisation further dental material is charged into the negative mould.

5. Method according to any of the claims 1 to 4, charachterised in that the dental prosthetic moulded parts comprising parts of dental prostheses, dental total prostheses, orthopedic prostheses or parts thereof, artificial teeth, veneers, inlays, onlays, superstructures, dental carrier structures, bridges, crowns, relinings, denture saddles, bone prostheses, joint prostheses, revision total joint endoprostheses and/or spacers.

6. Method according to any of the claims 1 to 4, **characterised in that** the dental material is, after mixing or when discharging and optionally mixing, directly charged from the cartridge into a negative mould or cuvette for the production of at least one dental prosthetic moulded part and polymerised.

## Revendications

1. Procédé de fabrication de pièces moulées prothétiques dentaires, comprenant les étapes suivantes :
- utilisation d'un matériau dentaire polymérisable prêt à l'emploi, dont la viscosité est comprise entre 5 000 mPa-s et 20 000 mPa-s de façon définie à une température ambiante d'environ 23 °C et comprenant un mélange de monomères présents sous forme d'une seule phase dans le mélange, sous forme d'au moins une pâte dans au moins une cartouche,
- déversement du matériau dentaire prêt à l'emploi, éventuellement en mélangeant le matériau dentaire, et
- introduction du matériau dentaire dans un moule négatif pour la fabrication d'au moins une pièce moulée prothétique dentaire, et
- en option, polymérisation du matériau dentaire,
la composition totale du matériau dentaire étant de 100 % en poids et le mélange comprenant
(1) de 25 à 90 % en poids d'au moins un diméthacrylate d'uréthane, ou d'un mélange comprenant au moins deux (méth)acrylates d'uréthane ;
(2) de 5 à 40 % en poids d'au moins un di(méth)acrylate exempt de groupes uréthanes, comprenant du diacrylate de néopentylglycol propoxylé, du diméthacrylate de triéthylèneglycol, du diméthacrylate de diéthylèneglycol, du di(méth)acrylate de décanediol, du di(méth)acrylate de dodécanediol, des di(méth)acrylates de bisphénol A éthoxylés/propoxylés, ou d'un mélange comprenant au moins deux di(méth)acrylates ;
(3) de 0 à 40 % en poids d'au moins un (méth)acrylate trifonctionnel, tétrafonctionnel ou suprafonctionnel, exempt de groupes uréthanes, ou d'un mélange comprenant au moins deux des (méth)acrylates précités, et
(4) de 0,1 à 60 % en poids de charge inorganique amorphe ou d'un mélange de charges inorganiques choisies parmi la silice pyrogénée ou une charge inorganique comprenant des silices de précipitation, des verres dentaires, des verres d'aluminosilicate, des verres de fluoroaluminosilicate, du silicate de baryum-aluminium, du silicate de strontium, du borosilicate de strontium, du silicate de lithium, du silicate de lithium-aluminium, des phyllosilicates, des zéolithes, des charges sphériques amorphes à base d'oxyde ou d'oxyde mixte, des fibres de verre et/ou fibres de carbone ainsi que des mélanges comprenant au moins l'une des charges précitées, et/ou
(5) de 0,01 à 5 % en poids d'un initiateur ou système d'initiateur pour la polymérisation à chaud.

2. Procédé selon la revendication 1, **caractérisé en ce que** le matériau dentaire se présente sous forme de pâte A et de pâte B séparées dans une cartouche ou dans deux cartouches et est mélangé lors du déversement.

3. Procédé selon la revendication 1 ou 2, **caractérisé en ce que** l'introduction dans le moule négatif se fait de façon pneumatique.

4. Procédé selon l'une des revendications 1 à 3, **caractérisé en ce que**, pendant la polymérisation, un autre matériau dentaire est introduit dans le moule négatif.

5. Procédé selon l'une des revendications 1 à 4, **caractérisé en ce que** les pièces moulées prothétiques dentaires comprennent des parties de prothèses dentaires, des prothèses dentaires totales, des prothèses orthopédiques ou des parties de celles-ci, des dents artificielles, des facettes, des inlays, des onlays, des superstructures, des structures de support dentaires, des bridges, des couronnes, des rebasages, des selles de prothèse, des prothèses osseuses, des prothèses articulaires, des endoprothèses articulaires totales de reprise et/ou des espaceurs.

6. Procédé selon l'une des revendications 1 à 4, **caractérisé en ce que** le matériau dentaire est introduit et polymérisé directement dans un moule négatif ou une cuvette pour la fabrication d'au moins une pièce moulée prothétique dentaire après le mélange ou lors du déversement et éventuellement du mélange hors de la cartouche.
